**Europäisches Patentamt**

(19) **European Patent Office**

**Office européen des brevets**

(11) Publication number : **0 277 821 B1**

(12) ## EUROPEAN PATENT SPECIFICATION

(45) Date of publication of patent specification :
**29.01.92 Bulletin 92/05**

(51) Int. Cl.[5] : **A61F 5/44**

(21) Application number : **88300899.7**

(22) Date of filing : **03.02.88**

(54) **Ostomy coupling.**

(30) Priority : **05.02.87 GB 8702608**

(43) Date of publication of application :
**10.08.88 Bulletin 88/32**

(45) Publication of the grant of the patent :
**29.01.92 Bulletin 92/05**

(84) Designated Contracting States :
**AT BE CH DE ES FR GR IT LI LU NL SE**

(56) References cited :
**EP-A- 0 068 778**
**EP-A- 0 089 138**

(56) References cited :
**DE-A- 3 417 183**
**DE-B- 1 266 929**
**GB-A- 1 579 875**
**US-A- 4 610 677**

(73) Proprietor : **E.R. Squibb & Sons, Inc.**
**Lawrenceville-Princeton Road**
**Princeton, N.J. 08540-4000 (US)**

(72) Inventor : **Steer, Peter Leslie**
**"Kingscote" Woodlands Rise**
**East Grinstead Sussex (GB)**

(74) Representative : **Cook, Anthony John et al**
**D. YOUNG & CO. 10, Staple Inn**
**London, WC1V 7RD (GB)**

EP 0 277 821 B1

## Description

The present invention relates to an ostomy coupling comprising a pair of coupling rings.

Ostomy couplings comprising a pair of coupling rings are known, and one commercially successful coupling of this kind is described and claimed in U.K. Patent No. 1 571 657. Other British Patents of interest are Nos. 1 568 860, 1 586 823, and 1 586 824.

The present invention aims at providing an ostomy coupling having improved security of connection between the coupling rings and which yet can be readily manipulated to separate the coupling rings. Separation of the coupling rings is necessary in order to change a used bag for a new bag.

According to the present invention, an ostomy coupling comprising a pair of coupling rings of which the body side ring has a radially extending flange attachable to a pad of medical grade adhesive and a chute wall surrounding a stomal aperture, and the bag side ring has a rib member of ring-like form integral with a seal strip, the rib member being received by a channel portion, also of ring-like form, of the body side ring when the rings are coupled together, the coupling being characterised in that the bag side ring has an integral flexible latching arm having a hook portion positioned to engage under a radially outwardly extending hook flange on the body side ring when the rings are coupled, the hook portion being disengageable from the hook flange by a lifting movement of the arm, and the bag side ring having, diametrically opposite to the latching arm, an integral flange to engage under the hook flange when the rings are coupled.

The invention will be better understood from the following description, given by way of example only, of an illustrative embodiment of the invention. This description is given with reference to the accompanying drawings in which:-

Figure 1 is an axial cross-section through one example of coupling according to the invention, Figures 1A and 1B respectively showing opposite ends of a diameter, and showing the body-side ring and the bag-side ring in their mutually connected condition;

Figure 2 is a front view on a reduced scale of a body-side coupling ring;

Figure 3 is an axial cross-section through the body-side coupling ring shown in Figure 2, taken on the line III-III; and

Figures 4 and 5 are respectively front view and axial sections of a bag-side ring usable in the present invention.

The body-side coupling ring 10 includes a radially extending flange 26 which is attachable to a pad 16 of a medical grade adhesive. The body-side ring 10 also has a cylindrical wall 24 surrounding the stomal aperture 14, this wall 24 serves as a chute to guide faecal discharges into an ostomy bag one wall of which is seen at 22. The pad 16 has a stomal aperture 18 therein, and, as is conventional, the medical grade adhesive pad 16 is attached to the wearer over the peristomal skin surface.

The illustrated coupling also includes a bag-side ring 12 to which the ostomy bag 22 is secured in any convenient manner. The ring 12 has an internally projecting deflectable peripheral sealing strip 20 which, in use, engages the radially outer surface of the wall 24. Reference is directed to U.K. Patent No. 1 568 860.

At one radial position on the bag side ring an integral flexible latching arm 30 joins the flange 28, this flange 28 having a hook portion 38 positioned to engage under a hook flange 40 on the body-side ring 10, in order to couple the two coupling rings together. The hook flange 40 is formed integrally with the remainder of the body-side ring 10, and extends completely around the flange 26. On the other hand, the latching hook portion 38 is located at one peripheral position only, although, depending on the relative orientation of the two coupling rings when the coupling is assembled, the hook portion 38 may engage under the flange 40 at any peripheral position. In other words, there is no necessity for the user when putting on a new bag, to adjust the rotary position of the bag side ring relative to the body side ring to any particular position, because the continuous peripheral structure of the hook flange 40 means that the user can couple the bag-side ring onto the body-side ring at any relative rotational orientation between the two rings.

To faciliate manufacture by injection moulding techniques, the flange 28 has a slot 36 therein, substantially aligned with the latching hook portion 38, through which a removable moulding tool can be inserted and withdrawn during manufacture.

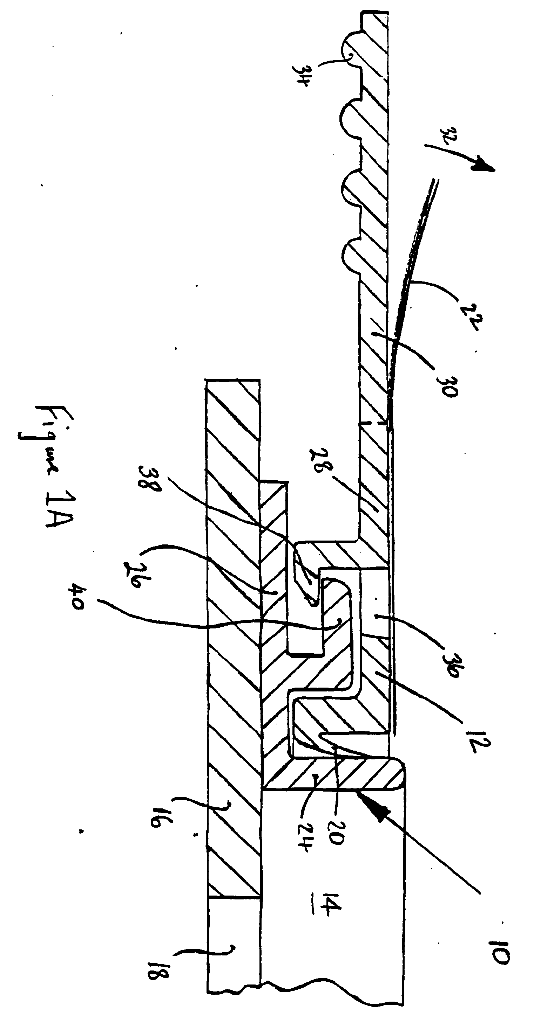

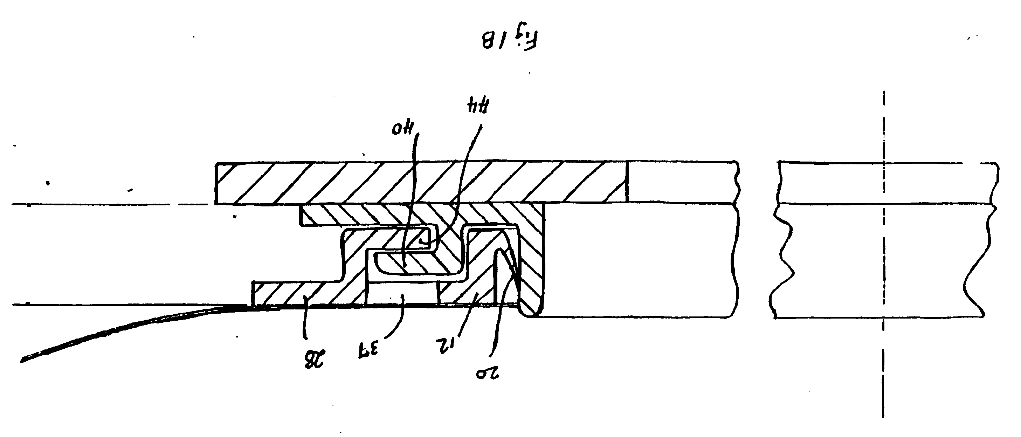

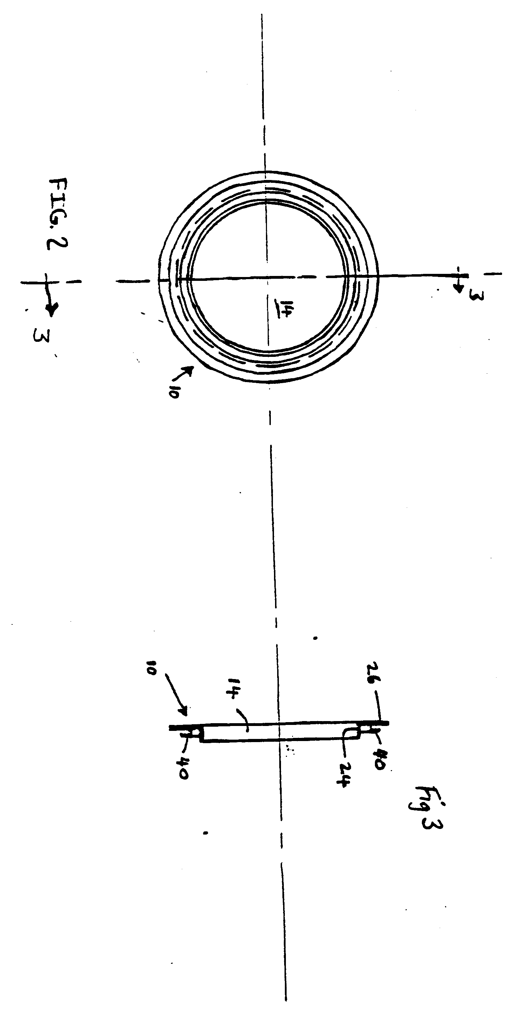

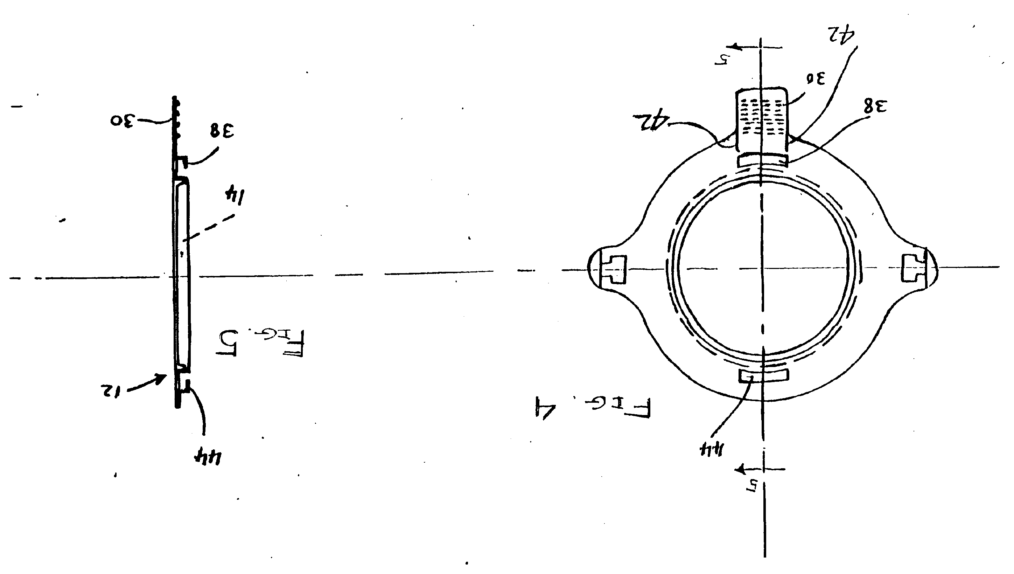

As an optional and not essential feature of the invention, the flexibility of the latching arm 30 relative to the remainder of the bag-side ring may be enhanced by the provision of relief slots through the flange 28. These slots may be located as shown at 42 on Figure 4, but are not shown in any other Figure. In certain circumstances no such slots may be necessary. As seen best in Figures 1 and 5, a ridged or bumped formation 34 may be employed for the latching arm, so that the user when he wishes to disengage the bag-side coupling part from the body-side coupling ring can conveniently pull the arm 30 in the direction indicated by the arrow 32, so causing the hook portion 38 to be disengaged from under the flange portion 40. Once this disengagement occurs, the bag-side ring and bag attached thereto can be readily disengaged so permitting the substitution of a clean bag.

On the bag-side ring, diametrically opposite to the latching arm 30, there is provided an integral flange 44 of limited peripheral extent. This flange 44 is made in one piece with the remainder of the bag-side coupling ring and an aperture 37 is provided to enable the flange 44 to be produced by injection moulding techniques. As seen in Figure 1B, in the coupled position of the two coupling rings, the flanges 40 and 44 are inter-engaged. Once the latching arm 30 has been lifted and the side shown in Figure 1A of the engaged coupling rings have been separated, then by a diametral motion to the right the flange 44 on the bag-side ring can be disengaged from the body-side ring which is then still on the wearer.

As will be understood by a man skilled in the art, the invention can be carried into effect in different ways. For example, while it is desirable that the hook flange 40 should extend completely around the body-side coupling, this is not essential. Other means of sealing than the flexible sealing strip 20 could be employed to effect a seal between the bag-side and body-side coupling rings.

## Claims

1. An ostomy coupling comprising a pair of coupling rings (10, 12) of which the body side ring (10) has a radially extending flange (26) attachable to a pad (16) of medical grade adhesive and a chute wall (24) surrounding a stomal aperture, and the bag side ring (12) has a rib member of ring-like form integral with a seal strip (20), the rib member being received by a channel portion, also of ring-like form, of the body side ring when the rings are coupled together, the coupling being characterised in that the bag side ring (12) has an integral flexible latching arm (30) having a hook portion (38) positioned to engage under a radially outwardly extending hook flange (40) on the body side ring (10) when the rings are coupled, the hook portion being disengageable from the hook flange by a lifting movement of the arm, and the bag side ring (12) having, diametrically opposite to the latching arm (30), an integral flange (44) to engage under the hook flange (40) when the rings are coupled.

2. An ostomy coupling according to claim 1 in which the hook flange (40) is peripherally continuous on the body side ring.

3. An ostomy coupling according to claim 1 or 2 in which the bag side member has a radially extending flange which has a slot (36) therein, substantially aligned with the latching hook portion (38), through which slot a removable moulding tool can be inserted and withdrawn during manufacture.

4. An ostomy coupling according to claim 1, 2 or 3 in which there are relief slots provided in the bag side ring to improve flexibility of the latching arm relative to the remainder of the bag side ring.

## Patentansprüche

1. Ostomiekupplung mit zwei Kupplungsringen (10, 12), von denen der körperseitige Ring (10) einen sich radial erstreckenden Flansch (26), der an einem Kissen (16) aus medizinischem Haftmittel anbringbar ist, und eine eine Stomaöffnung umgebende Rutschenwand (24) hat, und der beutelseitige Ring (12) ein ringförmiges Rippenelement hat, das mit einem Abdichtstreifen (20) einstückig ist, wobei das Rippenelement von einem ebenfalls ringförmigen Kanalabschnitt des körperseitigen Rings aufgenommen wird, wenn die Ringe miteinander verbunden sind, wobei die Kupplung dadurch gekennzeichnet ist, daß der beutelseitige Ring (12) einen einstückigen flexiblen Schnapparm (30) mit einem Hakenabschnitt (38) hat, der so angeordnet ist, daß er unter einem sich radial nach außen erstreckenden Hakenflansch (40) an dem körperseitigen Ring (10) in Eingriff kommt, wenn die Ringe verbunden sind, wobei der Hakenabschnitt von dem Hakenflansch durch eine Hebebewegung des Arms außer Eingriff bringbar ist, und der beutelseitige Ring (12) diametral gegenüberliegend zu dem Schnapparm (30) einen einstückigen Flansch (44) hat, der, wenn die Ringe verbunden sind, unter dem Hakenflansch (40) in Eingriff kommt.

2. Ostomiekupplung nach Anspruch 1, wobei der Hakenflansch (40) an dem körperseitigen Ring am Umfang durchgehend ist.

3. Ostomiekupplung nach Anspruch 1 oder 2, wobei das beutelseitige Element einen sich radial erstreckenden Flansch hat, der einen Schlitz (36) aufweist, der im wesentlichen mit dem Schnapphakenabschnitt (38) ausgerichtet ist, wobei durch diesen Schlitz bei der Herstellung ein entfernbares Gießwerkzeug eingesetzt und herausgenommen werden kann.

4. Ostomiekupplung nach Anspruch 1, 2 oder 3, wobei in dem beutelseitigen Ring Entlastungsschlitze vorgesehen sind, so daß die Flexibilität des Schnapparms in bezug auf den Rest des beutelseitigen Rings verbessert wird.

## Revendications

1. Raccord de stomie comprenant deux bagues de raccord (10,12) dont l'une (10), côté corps, possède une bride radiale (26) pouvant être fixée à un tampon (16) d'adhésif de qualité médicale et une paroi de goulotte (24) entourant une ouverture stomale (d'anus artificiel), et dont l'autre (12), côté sac, possède une nervure de forme annulaire faisant corps avec une bande d'étanchéité (20), la nervure venant se loger dans une partie creuse, elle aussi de forme annulaire, de la bague côté corps quand les bagues sont accouplées l'une à l'autre, ce raccord étant caractérisé en ce que la bague (12) côté sac possède un bras de blocage souple (30) faisant corps avec elle et ayant une partie crochet (38) disposée de façon à s'engager sous une bride de crochet (40), dirigée radialement vers l'extérieur, de la bague (10) côté corps quand les bagues sont accouplées, la partie crochet pouvant être dégagée de la bride de crochet par soulèvement du bras, et la bague (12) côté sac comportant, diamétralement à l'opposé du bras de blocage (30), une bride (44) faisant corps avec elle et destinée à s'engager sous la bride de crochet (40) quand les bagues sont accouplées.

2. Raccord de stomie selon la revendication 1, dans lequel la bride de crochet (40) est périphériquement continue sur la bague côté corps.

3. Raccord de stomie selon la revendication 1 ou 2, dans lequel l'élément côté sac comporte une bride radiale dans laquelle est pratiquée une fente (36), sensiblement en regard de la partie crochet de blocage ( 38), par laquelle un outil de moulage amovible peut être introduit et retiré en cours de fabrication.

4. Raccord de stomie selon la revendication 1, 2 ou 3, dans lequel des fentes de dégagement sont prévues dans la bague côté sac pour accroître la souplesse du bras de blocage par rapport au reste de la bague côté sac.

Figure 1A

EP 0 277 821 B1

Fig / B

20

12

44

40

37

28

FIG. 2

14

10

3

3

Fig 3

10

14

26

24

40

40

FIG. 4

FIG. 5